# EUROPEAN PATENT APPLICATION

(11) **EP 4 285 896 A1**
(43) Date of publication of application: **06.12.2023**
(21) Application number: 23174214.9
(22) Date of filing: 19.05.2023
(51) Int. Cl.: A61K 9/48, A61K 35/744, A61K 35/747, A61P 1/00, C12N 1/04, C12N 11/02, A61J 3/07

(54) **BANDING SOLUTION FOR MANUFACTURING ENTERIC HARD CAPSULES AND ENTERIC HARD CAPSULE MANUFACTURED USING THE SAME**

(30) Priority: 03.06.2022 KR 20220068153
(71) Applicant: Suheung Co., Ltd., Cheongju-si, Chungcheongbuk-do 28161 (KR)
(72) Inventor: YANG, Joo Hwan, 13449 Seongnam-si (KR)
(74) Representative: BCKIP Part mbB

(57) **Abstract**

The present invention relates to a banding solution for manufacturing enteric hard capsules and an enteric hard capsule manufactured using the same, and specifically, relates to banding solution for manufacturing enteric hard capsules, which prevent the generation of a gap between the coupled capsule upper portion and capsule lower portion due to gastric acid or the separation of the capsule upper portion and the capsule lower portion during the time the enteric hard capsules stay in the stomach, and an enteric hard capsule subjected to banding treatment using the same.

## Description

### CROSS-REFERENCE TO RELATED APPLICATION

This application claims priority to and the benefit of Korean Patent Application No. 10-2022-0068153, filed on June 3, 2022.

### BACKGROUND

### 1. Field of the Invention

The present invention relates to a banding solution for manufacturing enteric hard capsules and an enteric hard capsule manufactured using the same, and specifically, relates to banding solution for manufacturing enteric hard capsules, which prevent the generation of a gap between the coupled capsule upper portion and capsule lower portion due to gastric acid or the separation of the capsule upper portion and the capsule lower portion during the time the enteric hard capsules stay in the stomach, and an enteric hard capsule subjected to banding treatment using the same.

### 2. Discussion of Related Art

People take drugs, health functional foods, and the like for treatment of diseases, improvement of health, and the like. Pills are the most common formulation forms of drug or health functional food, the pills may be broadly divided into tablets and capsules, and capsules may be divided into soft capsules and hard capsules.

A tablet is a form in which a solidified drug is coated so that the drug can be dissolved and absorbed in the body. Soft capsules are generally manufactured using gelatin, and are manufactured by a method of putting an active ingredient, additives and the like between two sheets made of gelatin components and molding the sheets by applying pressure to the sheets. Therefore, a soft capsule is formed in one piece without separation or split gaps. A hard capsule has a shell in which the upper and lower portions are coupled, and is manufactured by a method of putting an active ingredient and an additive therein. In general, it is easier and less costly to manufacture hard capsules than to manufacture soft capsules.

Meanwhile, when not specially treated, pills are disintegrated by gastric acid during the time the pills remain in the stomach (within 2 hours) to release an active ingredient into the stomach, and as a result, the active ingredient is exposed to gastric acid and may be absorbed in the stomach. However, there is a need for a pill in a form whose active ingredient is inactivated by acid, is required to be absorbed in the intestines, or not disintegrated by gastric acid due to other requirements. Examples of such active ingredients include aspirin, lactic acid bacteria, omega-3, and the like. Aspirin may adversely affect patients with gastrointestinal diseases such as gastritis and stomach ulcers, and thus needs to be absorbed in the intestines, lactic acid bacteria should not be disintegrated by gastric acid because exposure to gastric acid reduces their effects, and in the case of omega-3, consumers prefer pills that are disintegrated in the intestines rather than the stomach due to its characteristic fishy odor. Therefore, there is a need for manufacturing enteric formulations, which are pills that are not disintegrated by gastric acid for 2 hours when the pills remain in the stomach and are disintegrated in the intestines within 30 or 60 minutes. As an enteric formulation in the related art, there are tablets coated with an enteric material and soft capsules consisting of the enteric material, and attempts have been made to manufacture enteric hard capsules due to the advantages of easy manufacturing and low cost.

However, enteric hard capsules attempted in the related art may have a structural problem in that even though the enteric hard capsule itself is not dissolved in gastric acid, hard capsule sites in contact with gastric acid swell because water-soluble components absorb moisture, whereas the inside of a coupling site of the hard capsule that does not come into direct contact with gastric acid does not swell, and as a result, a gap may occur at a coupling portion between the capsule upper portion and the capsule lower portion, or the capsule upper and lower portions may be separated to release the active ingredient.

### SUMMARY OF THE INVENTION

The present invention provides a banding solution for subjecting hard capsules to banding treatment to prevent the occurrence of a gap at a coupling portion between the capsule upper portion and the capsule lower portion or the separation between the capsule upper portion and the capsule lower portion due to gastric acid while the hard capsule stays in the stomach

The present invention provides an enteric hard capsule subjected to banding treatment, in which a gap at a coupling portion between the capsule upper portion and the capsule lower portion is prevented or the capsule upper portion and the capsule lower portion are not separated.

Provided is a banding solution which is applied to a region including a coupling portion of an enteric hard capsule for achieving the aforementioned objects.

In the present invention, the banding solution includes 90 to 96 parts by weight of purified water and 3 to 5 parts by weight of alginate.

In the present invention, one or more alginates are selected from the group consisting of sodium alginate, ammonium alginate, calcium alginate and potassium alginate.

In the present invention, the banding solution further includes 1 to 4 parts by weight of a corn starch-based polymer.

In the present invention, the banding solution further includes 1.5 to 2.5 parts by weight of pectin.

In the present invention, the banding solution further includes 1.5 to 2.5 parts by weight of pectin and 1.0 to 3.0 parts by weight of a corn starch-based polymer.

In the present invention, the banding solution includes 75 to 90 parts by weight of purified water, 2.5 to 6 parts by weight of pectin, 0.05 to 2 parts by weight of a plasticizer and 10 to 20 parts by weight of an alcohol.

In the present invention, the plasticizer is glycerin and the alcohol is ethanol.

In the present invention, the banding solution further includes 0.05 to 1.5 parts by weight of a colorant.

To achieve the aforementioned objects, provided is an enteric hard capsule applied to a region including a boundary of the coupling portion of the banding solution.

### BRIEF DESCRIPTION OF THE DRAWINGS

The above and other objects, features and advantages of the present invention will become more apparent to those of ordinary skill in the art by describing in detail exemplary embodiments thereof with reference to the accompanying drawings, in which:
FIG. 1 illustrates an enteric hard capsule and its configuration;
FIG. 2 illustrates enteric hard capsules obtained by applying a banding solution according to an exemplary embodiment and drying the banding solution;
FIG. 3 illustrates the results of performing a disintegration test under gastric acid conditions for 2 hours on enteric hard capsules obtained by applying a banding solution according to an exemplary embodiment and drying the banding solution; and
FIG. 4 illustrates an aspect in which a hard capsule to which a banding solution is not applied is subjected to a disintegration test under gastric acid conditions.

### DETAILED DESCRIPTION OF EXEMPLARY EMBODIMENTS

The present invention can be completely accomplished by the following description. The following description should be understood as describing specific embodiments of the present invention, and the present invention is not necessarily limited thereto. Further, it should be understood that the accompanying drawings are for the purpose of helping with understanding and that the present invention is not limited thereto.

Exemplary embodiments of the present disclosure are capable of various modifications and of being implemented in various forms. Therefore, it should be understood to include all modifications, equivalents or alternatives within a scope that the idea and technical features of the present disclosure are recognized as being identical.

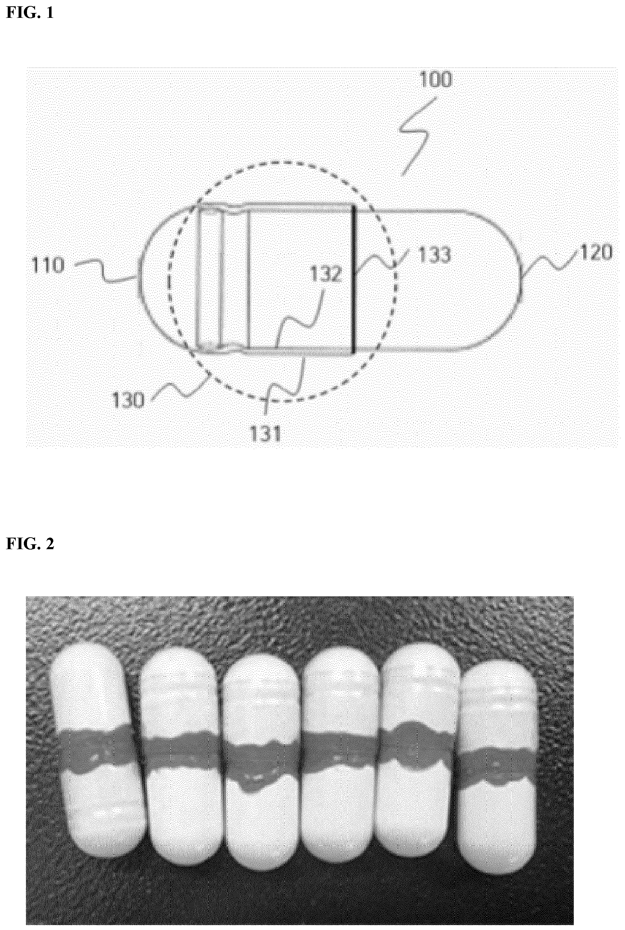

First, an enteric hard capsule according to the present invention will be described. FIG. 1 is a view schematically illustrating an enteric hard capsule 100 according to the present invention. Referring to FIG. 1, an enteric hard capsule 100 consists of a capsule upper portion 110 and a capsule lower portion 120, and the capsule upper portion 110 and the capsule lower portion 120 are coupled to form a coupling portion 130. The coupling portion 130 includes an outer coupling portion 131 which is exposed to the outside and corresponds to a part of the capsule upper portion 110 and an inner coupling portion 132 which is not exposed to the outside and corresponds to a part of the capsule lower portion. An active ingredient is present inside the enteric hard capsule 100. Examples of the active ingredient include lactic acid bacteria, omega-3, aspirin, agents for treating intestinal diseases, and the like, and are not limited thereto. The enteric hard capsule 100 is a hard capsule that is not disintegrated by gastric acid during the time it stays in the stomach (approximately 2 hours) and is disintegrated within 30 to 60 minutes in the intestines. A material for manufacturing an enteric hard capsule 100 may include at least one selected from the group consisting of hydroxypropyl methyl cellulose phthalate (HPMCP), cellulose acetate phthalate (CAP), hydroxypropyl methyl cellulose acetate succinate (HPMCAS), Eudragit, Zein, an acrylic acid copolymer, shellac, pectin, gellan gum, and alginate, and is not limited thereto.

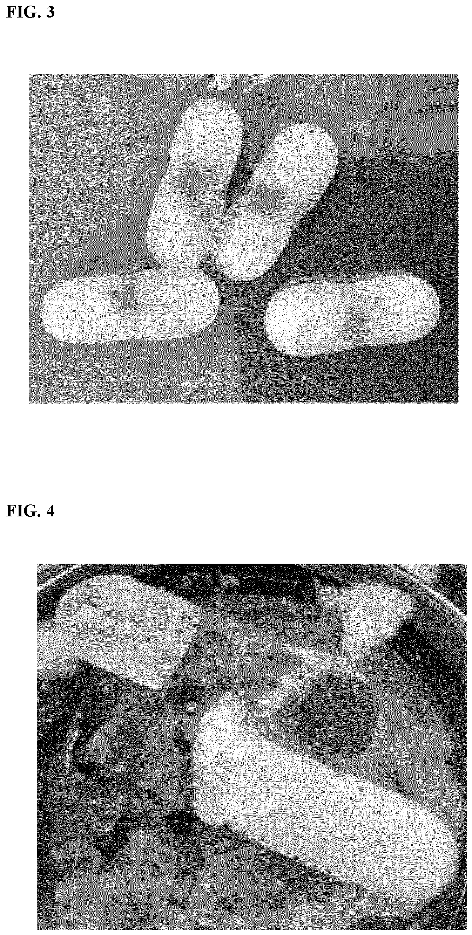

The enteric hard capsule 100 according to the present invention is characterized in that a banding solution is applied to a region including a part of the coupling portion 130 of the enteric hard capsule 100, more specifically a region including a boundary 133 of the coupling portion and dried. Unless such a banding solution is applied, the hard capsule itself is dissolved by gastric acid for 2 hours when the enteric hard capsule stays in the stomach, but the outer coupling portion 131 in contact with gastric acid swells as the water-soluble component absorbs moisture, whereas since the inner coupling portion 132, which does not come into direct contact with gastric acid, does not swell, there is a risk that a gap between the capsule upper portion and the capsule lower portion is generated or the capsule upper portion and the capsule lower portion are separated to release the active ingredient inside the hard capsule (FIG. 4). However, when a banding solution is applied to a region including a part of the coupling portion 130, a portion treated with the banding solution is brought into contact with gastric acid while the enteric hard capsule 100 remains in the stomach, so it is possible to prevent the external coupling portion of the part treated with the banding solution from absorbing water-soluble materials and swelling. Therefore, there is an advantage in that the active ingredient is not released due to the generation of a gap between the capsule upper portion and the capsule lower portion and the separation between the capsule upper portion and the capsule lower portion. In addition, since the banding solution is applied only to a region including the coupling portion 130, more specifically, a region including the boundary 133 of the coupling portion, the enteric hard capsule of the present invention is more economical than applying the banding solution to the entire hard capsule, and is easily transported and ingested because an increase in volume is small. Furthermore, after the hard capsule is manufactured, the banding solution may be applied to the hard capsule and dried, so the application and drying processes of the banding solution may be easily added to the existing manufacturing process.

Hereinafter, a banding solution according to an exemplary embodiment of the present invention will be described.

The banding solution according to an exemplary embodiment of the present invention includes 90 to 96 parts by weight of distilled water and 3 to 5 parts by weight of alginate.

Alginate may complement the acid resistance of the enteric hard capsule 100 by increasing the viscosity of the banding solution and enhancing the acid resistance of the banding solution. As the alginate, one or more may be selected from the group consisting of sodium alginate, ammonium alginate, calcium alginate and potassium alginate.

Alginic acid of the alginate is a polymer compound derived from brown algae, and a weakly acidic uronic acid polymer into which a carboxylic group (-COOH, -CO₂H) is introduced instead of a primary alcohol group of aldose by forming a long chain covalent bond between β-1,4-D-mannuronic acid and α-1,4-L-guluronic acid.

Meanwhile, it is very important for the banding solution to have an optimum viscosity for manufacturing and smooth application. In this regard, the inventors of the present invention conducted numerous experiments, and as a result, could not solve a problem that when the content of alginate was less than 3 parts by weight, it was not sufficient to have a required acid resistance, so a gap at the boundary of a coupling portion was generated, or the capsule upper portion and the capsule lower portion were separated even though enteric hard capsules were manufactured using a banding solution. In addition, since the banding solution was thin, even though the banding solution was applied to the hard capsules, a phenomenon in which the banding solution flowed occurred, so it was difficult to apply the banding solution to a desired region, causing a problem in product manufacturing. In contrast, it was confirmed that when the content of alginate exceeded 5 parts by weight, the viscosity became excessively high, and thus, there was a problem in that the banding solution did not smoothly move in a machine device, or the banding solution was not applied to a desired region even though it was applied to hard capsules by hand and the like, and there was a problem in product manufacturing because the banding solution was not well dried even though the banding solution was applied.

The banding solution according to an exemplary embodiment of the present invention may further include a corn starch-based polymer in addition to purified water and alginate.

The corn starch-based polymer may complement the acid resistance of the enteric hard capsule 100. As the corn starch-based polymer, commercially available products may be used, and for example, Eudraguard^{®} natural manufactured by Evonik Industries AG may be used.

In an exemplary embodiment including the corn starch-based polymer, the banding solution may include 90 to 96 parts by weight of distilled water, 3 to 5 parts by weight of alginate and 1 to 4 parts by weight of the corn starch-based polymer.

The banding solution according to an exemplary embodiment of the present invention may further include pectin in addition to distilled water and alginate. Pectin increases the viscosity of the banding solution and may enhance the acid resistance of the banding solution.

Pectin is a water-soluble polymer polysaccharide obtained by extracting edible plants such as citrus or apples. According to the degree of esterification (DE), pectin with a DE of 50% or more is called high methoxyl (HM) pectin, and that with a DE of less than 50% is called low methoxyl (LM) pectin. In addition, pectin obtained by treating LM pectin with ammonia under alkaline conditions is called LM amide pectin. In this case, a pectin used in the banding solution according to an exemplary embodiment of the present invention may be LM pectin or LM amide pectin.

In an exemplary embodiment including pectin, the banding solution may include 90 to 96 parts by weight of distilled water, 3 to 5 parts by weight of alginate and 1.5 to 2.5 parts by weight of pectin.

The banding solution according to an exemplary embodiment of the present invention may further include a corn starch-based polymer and pectin in addition to purified water and alginate.

In an exemplary embodiment including the corn starch-based polymer and pectin, the banding solution may include 90 to 96 parts by weight of distilled water, 3 to 5 parts by weight of alginate, 1.5 to 2.5 parts by weight of pectin and 1.0 to 3.0 parts by weight of the corn starch-based polymer.

Meanwhile, the banding solution according to an exemplary embodiment of the present invention may further include a colorant. The colorant may be included in the banding solution in an amount of 0.05 to 1.5 parts by weight. When a colorant is included, a colored banding solution may be prepared, and a portion to which the banding solution is applied may be made visible or may be prepared in the same color as the hard capsule. As the colorant, it is possible to use one or more dyes selected from the group consisting of Green No. 3, Red No. 2, Red No. 3, Red No. 40, Red No. 102, Blue No. 1, Blue No. 2, Yellow No. 4, Yellow No. 5 and Aluminum Lake, and the colorant is not limited thereto.

Hereinafter, a banding solution according to another exemplary embodiment of the present invention will be described.

The banding solution according to another exemplary embodiment of the present invention may include 75 to 85 parts by weight of distilled water, 2.5 to 6 parts by weight of pectin, 0.05 to 2 parts by weight of a plasticizer and 10 to 20 parts by weight of an alcohol.

Pectin may complement the acid resistance of the enteric hard capsule 100 by increasing the viscosity of the banding solution and enhancing the acid resistance of the banding solution.

Meanwhile, it is very important for the banding solution to have an optimum viscosity for manufacturing and smooth application. In this regard, the inventors of the present invention conducted numerous experiments, and as a result, could not solve a problem that when the content of pectin was less than 2.5 parts by weight, it was not sufficient to have a required acid resistance, so a gap at the boundary of a coupling portion was generated, or the capsule upper portion and the capsule lower portion were separated even though enteric hard capsules were manufactured using a banding solution. In addition, since the banding solution was thin, even though the banding solution was applied to the hard capsules, a phenomenon in which the banding solution flowed occurred, so it was difficult to apply the banding solution to a desired region, causing a problem in product manufacturing. In contrast, it was confirmed that when the content of pectin exceeded 6 parts by weight, the viscosity became excessively high, and thus, there was a problem in that the banding solution did not smoothly move in a machine device, or the banding solution was not applied to a desired region even though it was applied to hard capsules by hand and the like, and there was a problem in product manufacturing because the banding solution was not well dried even though the banding solution was applied.

The plasticizer may improve the processability of the banding solution. Examples of the plasticizer include glycerin, glycerin fatty acid esters, dioctyl adipate, polyester adipates, epoxidized soybean oil, epoxyhexahydrophthalate diesters, kaolin and triethyl citrate, and are not limited thereto.

The alcohol helps dissolve the components of the banding solution and enables quick drying after the coating process of the banding solution. Examples of alcohols include ethanol, methanol, isopropanol and butanol, and are not limited thereto.

Meanwhile, the banding solution according to another exemplary embodiment of the present invention may further include a colorant. The colorant may be included in the banding solution in an amount of 0.05 to 1.5 parts by weight. When a colorant is included, a colored banding solution may be prepared, and a portion to which the banding solution is applied may be made visible or may be prepared in the same color as the hard capsule. As the colorant, it is possible to use one or more dyes selected from the group consisting of Green No. 3, Red No. 2, Red No. 3, Red No. 40, Red No. 102, Blue No. 1, Blue No. 2, Yellow No. 4, Yellow No. 5 and Aluminum Lake, and the colorant is not limited thereto.

The enteric hard capsule according to an exemplary embodiment of the present invention is manufactured by a step of applying a banding solution to a region including a coupling portion 130 of a hard capsule having acid resistance, more specifically, a region including a boundary 133 of the coupling portion and a step of drying the banding solution. A hard capsule having acid resistance may be manufactured by a typical manufacturing method.

The present disclosure will be described in more detail through the following Examples and Comparative Examples. However, the present disclosure is not limited by the Examples.

### Experimental Example

An acid-resistant hard capsule (SUHEUNG EMBOCAPS-AP, No. 0) filled with lactic acid bacteria was subjected to a disintegration test under gastric acid (pH 1.2) conditions for 1 hour. FIG. 4 illustrates an aspect in which a hard capsule to which a banding solution is not applied is subjected to a disintegration test under gastric acid conditions. As a result of the disintegration test, it was confirmed that the capsule upper part and the capsule lower part of the hard capsule were separated to release a preparation inside the capsule.

### Examples 1 and 2

Sodium alginate was added to 70°C purified water and the resulting mixture was stirred. Thereafter, a dye (Blue No. 1) was added thereto and the resulting mixture was stirred. It was confirmed that when the prepared banding solution was cooled at room temperature, the banding solution was well mixed without layer separation. The prepared banding solution was manually applied to an acid-resistant hard capsule (SUHEUNG EMBOCAPS-AP, No. 0) filled with lactic acid bacteria and then dried. As a result of observing the manufactured enteric hard capsule after the disintegration test under gastric acid (pH 1.2) conditions for 2 hours, it was confirmed that the capsule upper portion and the capsule lower portion of the hard capsule were not separated, and as a result of a subsequent disintegration test in distilled water after the disintegration test under gastric acid conditions, it was confirmed that the enteric hard capsule was disintegrated within 30 minutes.

### Comparative Examples 1 and 2

Sodium alginate was added to 70°C purified water and the resulting mixture was stirred. Thereafter, a dye (Blue No. 1) was added thereto and the resulting mixture was stirred. When the prepared banding solution was cooled at room temperature, the disintegration test could not be performed because it was impossible to apply the banding solution due to low or high viscosity.

**Table 1**

| | **Example 1** | **Example 2** | **Comparative Example 1** | **Comparative Example 2** |
|---|---|---|---|---|
| Purified water (wt%) | 94.90 | 95.90 | 89.90 | 97.40 |
| Sodium Alginate | 5.00 | 4.00 | 10.00 | 2.50 |
| (wt%) | | | | |
| Blue No. 1 (wt%) | 0.10 | 0.10 | 0.10 | 0.10 |
| Sum (wt%) | 100.00 | 100.00 | 100.00 | 100.00 |
| **Gastric acid disintegration (pH 1.2, 2 hrs or more)** | **Suitable** | **Suitable** | **Experiment is not possible** | **Experiment is not possible** |
| **Purified water disintegration (within 30 min)** | **Suitable** | **Suitable** | **Experiment is not possible** | **Experiment is not possible** |
| **Features** | **Good viscosity Banding is possible** | **Good viscosity Banding is possible** | **High viscosity Banding is not possible** | **Low viscosity Banding is not possible** |

### Examples 3 and 4

Sodium alginate and Eudraguard^{®} natural were added to 70°C purified water, and the resulting mixture was stirred. Thereafter, a dye (Blue No. 1) was added thereto and the resulting mixture was stirred. It was confirmed that when the prepared banding solution was cooled at room temperature, the banding solution was well mixed without layer separation. The prepared banding solution was manually applied to an acid-resistant hard capsule (SUHEUNG EMBOCAPS-AP, No. 0) filled with lactic acid bacteria and then dried. As a result of observing the manufactured enteric hard capsule after the disintegration test under gastric acid (pH 1.2) conditions for 2 hours, it was confirmed that the capsule upper portion and the capsule lower portion of the hard capsule were not separated, and as a result of a subsequent disintegration test in distilled water after the disintegration test under gastric acid conditions, it was confirmed that the enteric hard capsule was disintegrated within 30 minutes.

### Comparative Examples 3 and 4

Sodium alginate and Eudraguard^{®} natural were added to 70°C purified water, and the resulting mixture was stirred. Thereafter, a dye (Blue No. 1) was added thereto and the resulting mixture was stirred. When the prepared banding solution was cooled at room temperature, the disintegration test could not be performed because it was impossible to apply the banding solution due to low or high viscosity.

**Table 2**

| | **Example 3** | **Example 4** | **Comparative Example 3** | **Comparative Example 4** |
|---|---|---|---|---|
| Purified water (wt%) | 93.40 | 91.40 | 96.90 | 87.40 |
| Sodium Alginate (wt%) | 3.25 | 5.00 | 2.50 | 6.25 |
| Eudraguard natural (wt%) | 3.25 | 3.50 | 0.50 | 6.25 |
| Blue No. 1 (wt%) | 0.10 | 0.10 | 0.10 | 0.10 |
| Sum (wt%) | 100.00 | 100.00 | 100.00 | 100.00 |
| **Gastric acid disintegration (pH 1.2, 2 hrs or more)** | **Suitable** | **Suitable** | **Experiment is not possible** | **Experiment is not possible** |
| **Purified water disintegration (within 30 min)** | **Suitable** | **Suitable** | **Experiment is not possible** | **Experiment is not possible** |
| **Features** | **Good viscosity Banding is possible** | **Good viscosity Banding is possible** | **Low viscosity Banding is not possible** | **High viscosity Banding is not possible** |

### Examples 5 and 6

Sodium alginate and LM amide pectin were added to 70°C purified water, and the resulting mixture was stirred. Thereafter, a dye (Blue No. 1) was added thereto and the resulting mixture was stirred. It was confirmed that when the prepared banding solution was cooled at room temperature, the banding solution was well mixed without layer separation. The prepared banding solution was manually applied to an acid-resistant hard capsule (SUHEUNG EMBOCAPS-AP, No. 0) filled with lactic acid bacteria and then dried. As a result of observing the manufactured enteric hard capsule after the disintegration test under gastric acid (pH 1.2) conditions for 2 hours, it was confirmed that the capsule upper portion and the capsule lower portion of the hard capsule were not separated, and as a result of a subsequent disintegration test in distilled water after the disintegration test under gastric acid conditions, it was confirmed that the enteric hard capsule was disintegrated within 30 minutes.

### Comparative Examples 5 and 6

Sodium alginate and LM amide pectin were added to 70°C purified water, and the resulting mixture was stirred. Thereafter, a dye (Blue No. 1) was added thereto and the resulting mixture was stirred. When the prepared banding solution was cooled at room temperature, the disintegration test could not be performed because it was impossible to apply the banding solution due to low or high viscosity.

**Table 3**

| | **Example 5** | **Example 6** | **Comparative Example 5** | **Comparative Example 6** |
|---|---|---|---|---|
| Purified water (wt%) | 94.65 | 92.40 | 96.40 | 90.65 |
| Sodium Alginate (wt%) | 3.25 | 5.00 | 2.50 | 6.25 |
| Pectin (wt%) | 2.00 | 2.50 | 1.00 | 3.00 |
| Blue No. 1 (wt%) | 0.10 | 0.10 | 0.10 | 0.10 |
| Sum (wt%) | 100.00 | 100.00 | 100.00 | 100.00 |
| **Gastric acid disintegration (pH 1.2, 2 hrs or more)** | **Suitable** | **Suitable** | **Experiment is not possible** | **Experiment is not possible** |
| **Purified water disintegration (within 30 min)** | **Suitable** | **Suitable** | **Experiment is not possible** | **Experiment is not possible** |
| **Features** | **Good viscosity Banding is possible** | **Good viscosity Banding is possible** | **Low viscosity Banding is not possible** | **High viscosity Banding is not possible** |

### Examples 7 and 8

Sodium alginate, LM amide pectin and Eudraguard^{®} natural were added to 70°C purified water, and the resulting mixture was stirred. Thereafter, a dye (Blue No. 1) was added thereto and the resulting mixture was stirred. It was confirmed that when the prepared banding solution was cooled at room temperature, the banding solution was well mixed without layer separation. The prepared banding solution was manually applied to an acid-resistant hard capsule (SUHEUNG EMBOCAPS-AP, No. 0) filled with lactic acid bacteria and then dried. As a result of observing the manufactured enteric hard capsule after the disintegration test under gastric acid (pH 1.2) conditions for 2 hours, it was confirmed that the capsule upper portion and the capsule lower portion of the hard capsule were not separated, and as a result of a subsequent disintegration test in distilled water after the disintegration test under gastric acid conditions, it was confirmed that the enteric hard capsule was disintegrated within 30 minutes.

### Comparative Examples 7 and 8

Sodium alginate, LM amide pectin and Eudraguard^{®} natural were added to 70°C purified water, and the resulting mixture was stirred. Thereafter, a dye (Blue No. 1) was added thereto and the resulting mixture was stirred. When the prepared banding solution was cooled at room temperature, the disintegration test could not be performed because it was impossible to apply the banding solution due to low or high viscosity.

**Table 4**

| | **Example 7** | **Example 8** | **Comparative Example 7** | **Comparative Example 8** |
|---|---|---|---|---|
| Purified water (wt%) | 93.65 | 90.40 | 96.40 | 88.65 |
| Sodium Alginate (wt%) | 3.25 | 4.50 | 2.00 | 6.25 |
| Pectin (wt%) | 2.00 | 2.00 | 1.00 | 4.00 |
| Eudraguard natural (wt%) | 1.00 | 3.00 | 0.50 | 1.00 |
| Blue No. 1 (wt%) | 0.10 | 0.10 | 0.10 | 0.10 |
| Sum (wt%) | 100.00 | 100.00 | 100.00 | 100.00 |
| **Gastric acid disintegration (pH 1.2, 2 hrs or more)** | **Suitable** | **Suitable** | **Experiment is not possible** | **Experiment is not possible** |
| **Purified water disintegration (within 30 min)** | **Suitable** | **Suitable** | **Experiment is not possible** | **Experiment is not possible** |
| **Features** | **Good viscosity Banding is possible** | **Good viscosity Banding is possible** | **Low viscosity Banding is not possible** | **High viscosity Banding is not possible** |

### Examples 9 and 10

LM amide pectin was added to 70°C purified water, and the resulting mixture was stirred. Further, glycerin was added thereto, the resulting mixture was stirred, and a dye (Blue No. 1) was added thereto. Thereafter, while maintaining the temperature at 60°C, ethanol was added thereto and the resulting mixture was stirred. It was confirmed that when the prepared banding solution was cooled at room temperature, the banding solution was well mixed without layer separation. The prepared banding solution was manually applied to an acid-resistant hard capsule (SUHEUNG EMBOCAPS-AP, No. 0) filled with lactic acid bacteria and then dried. As a result of observing the manufactured enteric hard capsule after the disintegration test under gastric acid (pH 1.2) conditions for 2 hours, it was confirmed that the capsule upper portion and the capsule lower portion of the hard capsule were not separated, and as a result of a subsequent disintegration test in distilled water after the disintegration test under gastric acid conditions, it was confirmed that the enteric hard capsule was disintegrated within 30 minutes.

### Comparative Examples 9 and 10

LM amide pectin was added to 70°C purified water, and the resulting mixture was stirred. Further, glycerin was added thereto, the resulting mixture was stirred, and a dye (Blue No. 1) was added thereto. Thereafter, while maintaining the temperature at 60°C, ethanol was added thereto and the resulting mixture was stirred. When the prepared banding solution was cooled at room temperature, the disintegration test could not be performed because it was impossible to apply the banding solution due to low or high viscosity.

**Table 5**

| | **Example 9** | **Example 10** | **Comparative Example 9** | **Comparative Example 10** |
|---|---|---|---|---|
| Purified water (wt%) | 80.90 | 79.90 | 81.90 | 75.90 |
| Pectin (wt%) | 3.00 | 4.00 | 2.00 | 8.00 |
| Glycerin (wt%) | 1.00 | 1.00 | 1.00 | 1.00 |
| EtOH (wt%) | 15.00 | 15.00 | 15.00 | 15.00 |
| Blue No. 1 (wt%) | 0.10 | 0.10 | 0.10 | 0.10 |
| Sum (wt%) | 100.00 | 100.00 | 100.00 | 100.00 |
| **Gastric acid disintegration (pH 1.2, 2 hrs or more)** | **Suitable** | **Suitable** | **Experiment is not possible** | **Experiment is not possible** |
| **Purified water disintegration (within 30 min)** | **Suitable** | **Suitable** | **Experiment is not possible** | **Experiment is not possible** |
| **Features** | **Good viscosity Banding is possible** | **Good viscosity Banding is possible** | **Low viscosity Banding is not possible** | **High viscosity Banding is not possible** |

According to the present invention, it is possible to manufacture a banding solution for manufacturing an enteric capsule that prevents a gap at the boundary of the coupling portion due to gastric acid or a separation of the capsule upper portion and the capsule lower portion for 2 hours when a hard capsule stays in the stomach after ingestion, thereby preventing the release of an active ingredient.

According to the present invention, an enteric hard capsule can be manufactured by applying and drying the banding solution.

## Claims

1. A banding solution applied to a region comprising a boundary of a coupling portion of an enteric hard capsule in which a capsule upper portion and a capsule lower portion are coupled to form the coupling portion,
wherein the banding solution comprises 90 to 96 parts by weight of purified water and 3 to 5 parts by weight of alginate.

2. The banding solution of claim 1, wherein the alginate is one or more selected from the group consisting of sodium alginate, ammonium alginate, calcium alginate and potassium alginate.

3. The banding solution of claim 1 or 2, further comprising 1.0 to 4.0 parts by weight of a corn starch-based polymer.

4. The banding solution of any one of claims 1 to 3, further comprising 1.5 to 2.5 parts by weight of pectin.

5. The banding solution of any one of claims 1 to 4, further comprising 1.5 to 2.5 parts by weight of pectin and 1.0 to 3.0 parts by weight of a corn starch-based polymer.

6. A banding solution applied to a region comprising a boundary of a coupling portion of an enteric hard capsule in which a capsule upper portion and a capsule lower portion are coupled to form the coupling portion,
wherein the banding solution comprises 75 to 85 parts by weight of distilled water, 2.5 to 6 parts by weight of pectin, 0.05 to 2 parts by weight of a plasticizer and 10 to 20 parts by weight of an alcohol.

7. The banding solution of claim 6, wherein the plasticizer is glycerin, and
the alcohol is ethanol.

8. The banding solution of any one of claims 1 to 7, further comprising 0.05 to 1 part by weight of a colorant.

9. An enteric hard capsule with the banding solution of any one of claims 1 to 8 applied to a region comprising a boundary of a coupling portion.
